# NEUE EUROPÄISCHE PATENTSCHRIFT

(11) **EP 1 809 243 B2**
(45) Veröffentlichungstag und Bekanntmachung des Hinweises auf die Entscheidung über den Einspruch: **08.06.2022**
(45) Hinweis auf die Patenterteilung: 18.07.2012
(21) Anmeldenummer: 05761637.7
(22) Anmeldetag: 25.06.2005
(51) Int. Cl.: A61K 9/12, A61K 31/46, A61P 11/06

(54) **AEROSOLSUSPENSIONSFORMULIERUNGEN MIT TG 227 EA ALS TREIBMITTEL**
AEROSOL SUSPENSION FORMULATIONS CONTAINING TG 227 EA AS A PROPELLANT
FORMULATIONS DE SUSPENSIONS AEROSOL CONTENANT TG 227 EA EN TANT QU'AGENT PROPULSEUR

(30) Priorität: 02.07.2004 DE 102004032322; 17.05.2005 DE 102005023334
(43) Veröffentlichungstag der Anmeldung: 25.07.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: SCHMELZER, Christel, 55218 INGELHEIM (DE); FROEMDER, Arne, 55218 INGELHEIM (DE)
(86) Internationale Anmeldenummer: PCT/EP2005/006865
(87) Internationale Veröffentlichungsnummer: WO 2006/002840

(56) Entgegenhaltungen:
- EP-A- 1 527 772
- EP-A1- 1 527 772
- WO-A-02/05785
- WO-A-93/15741
- WO-A-2004/084858
- WO-A1-00/33892
- WO-A1-00/33892
- WO-A1-02/05785
- WO-A1-93/15741
- WO-A1-03/082252
- AU-A1- 2003 209 743
- US-A- 5 955 058
- US-A- 5 955 958
- WILLIAMS R O ET AL: "INFLUENCE OF METERING CHAMBER VOLUME AND WATER LEVEL ON THE EMITTED DOSE OF A SUSPENSION-BASED PDMI CONTAINING PROPELLANT 134A" PHARMACEUTICAL RESEARCH, NEW YORK, NY, US, Bd. 14, Nr. 4, 1997, Seiten 438-443, XP002907568 ISSN: 0724-8741

## Beschreibung

Die Erfindung betrifft Druckgaszubereitungen für Dosieraerosole bei denen ein Arzneimittel in TG 227 ea (1,1,1,2,3,3,3-Heptafluorpropan) als Treibmittel suspendiert formuliert ist, sowie deren Verwendung zur Herstellung eines Arzneimittels. Bevorzugt handelt es sich um ein Inhalationsaerosol.

### Stand der Technik

Seit der öffentlichen Diskussion um das ozonschädigende Potential bestimmter Fluorchlorkohlenwasserstoffe wird intensiv auf dem Gebiet von alternativen Treibgasen für pharmazeutische Dosieraerosole gearbeitet. Seit Anfang der 1990er Jahre ist bekannt, dass die Treibgase TG227 ea oder TG134 a als alternative Treibgase für Aerosole eingesetzt werden können.

Überraschend hat sich nun herausgestellt, dass Treibgasformulierungen mit suspendierten Wirkstoffpartikeln und TG 227 ea als Treibmittel stabilisiert werden können, wenn das Treibgas oder Treibgasgemisch etwas Wasser enthält.

### Detaillierte Beschreibung der Erfindung

Für die erfindungsgemäßen Treibmittelformulierungen wird als Treibgas TG 227 ea eingesetzt, gegebenenfalls in Mischung mit einem oder mehreren weiteren Treibgasen, vorzugsweise ausgewählt aus der Gruppe bestehend aus Propan, Butan, Pentan, Dimethylether, CHClF₂, CH₂F₂, CF₃CH₃, Isobutan, Isopentan und Neopentan.

Erfindungsgemäß bevorzugt sind solche Suspensionen, die als Treibgas nur TG 227 ea enthalten.
In Gemischen mit einem oder mehreren weiteren Treibgasen, ausgewählt aus der Gruppe bestehend aus Propan, Butan, Pentan, Dimethylether, CHClF₂, CH₂F₂, CF₃CH₃, Isobutan, Isopentan und Neopentan eingesetzt, liegt der Anteil dieser weiteren Treibgaskomponente vorzugsweise unter 60 %, bevorzugt unter 40% besonders bevorzugt unter 30%.
Als Wirkstoffe mit chemisch gebundenem Wasser werden bevorzugt Wirkstoffe eingesetzt, die ein oder mehrere Wassermoleküle in ihrer Partikelstruktur einlagern oder binden. Dabei ist das Wasser nicht lediglich physikalisch mit den Wirkstoffpartikeln vermengt. Bevorzugt handelt es sich bei den Wirkstoffpartikeln um Kristalle und bei dem Wasser um Kristallwasser oder komplex-gebundenes Wasser oder anderweitig chemisch gebundenes Wasser, z.B. Hydrate. Diese Form der Wassereinlagerung wird im Folgenden auch chemisch gebundenes Wasser genannt. In diesen Fällen hat das Wasser in Regel auch Einfluss auf die Kristallstruktur.

Die erfindungsgemäßen Formulierungen enthalten Ipratropiumbromid-Monohydrat oder Tiotropiumbromid-Monohydrat.

Für die Inhalation kommen Arzneimittel mit den o.g. Wirkstoffen mit chemisch gebundenem Wasser in Betracht, sowie deren Salze, Ester sowie die Kombination dieser Wirkstoffe, Salze und Ester.

Der Anteil des suspendierten Arzneistoffs an der fertigen Zubereitung beträgt zwischen 0,001 und 5 %, vorzugsweise 0,005 bis 3 %, insbesondere 0,01 bis 2 %. Oberflächenaktive-Stoffe werden in Mengen von 0,01 bis 10 %, vorzugsweise 0,05 bis 5 %, insbesondere 0,05
bis 3 % zugegeben (% = Gewichtsprozent).

Im Fall von Ipratropiumbromid-Monohydrat enthalten die erfindungsgemäßen Suspensionen vorzugsweise zwischen 0,001 bis 1%, besonders 0,005 bis 0,5 % Ipratropium. Erfindungsgemäß besonders bevorzugt sind Suspensionen, die 0,01 bis 0,1% Ipratropium enthalten.

Im Fall von Tiotropiumbromid-Monohydrat enthalten die erfindungsgemäßen Suspensionen vorzugsweise zwischen 0,001 bis 1%, besonders 0,0012 bis 0,8% Tiotropium. Erfindungsgemäß bevorzugt sind Suspensionen, die 0,002 bis 0,5 %, besonders bevorzugt 0,008 bis 0,4 % Tiotropium enthalten.

Unter Tiotropium oder Ipratropium, ist jeweils das freie Ammoniumkation zu verstehen. Die erfindungsgemäßen Treibgas-Suspensionen sind dadurch gekennzeichnet, dass sie Tiotropium oder Ipratropium in Form der kristallinen Monohydrate enthalten. Dementsprechend betrifft die vorliegende Erfindung Suspensionen, die kristallines Tiotropiumbromid-Monohydrat oder Ipratropiumbromid-Monohydrat enthalten. In Bezug auf Tiotropiumbromid-Monohydrat sind Suspensionen von besonderem Interesse, die 0,001 bis 0,62%, besonders bevorzugt 0,002 bis 0,5%, ganz besonders bevorzugt 0,002 bis 0,06 kristallines Tiotropiumbromid-Monohydrat enthalten.

Bei den im Rahmen der vorliegenden Erfindung genannten prozentualen Angaben, handelt es sich stets um Massenprozente. Werden Massenanteile für Tiotropium in Massenprozenten zum Ausdruck gebracht, sind die entsprechenden Werte für das im Rahmen der vorliegenden Erfindung bevorzugt zum Einsatz gelangende kristalline Tiotropiumbromid-monohydrat durch Multiplikation mit dem Umrechnungsfaktor 1,2495 erhältlich. Analoges gilt für Ipratopium.

Werden wasserfreie Treibgase eingesetzt, werden diesen erfindungsgemäß eine geringe Menge an Wasser beigemischt. Erfindungsgemäß können aber auch wasserhaltige Treibgase eingesetzt werden, wobei diese bei ihrer Verwendung einen bestimmten Wassergehalt aufweisen sollten. Dieses zugesetzte bzw. vorhandene Wasser ist in der fertigen Supensionsformulierung verschieden von Wasser, welches in einem der Wirkstoffe oder den Hilfsstoffen chemisch gebunden vorliegt. Im Rahmen der vorliegenden Erfindung wird dieses nicht chemisch gebundene Wasser auch als freies Wasser bezeichnet, um es von dem mit dem Wirkstoff molekular bzw. chemisch verbundenem Wasser abzugrenzen.

Es hat sich gezeigt, dass sich die suspendierten Wirkstoffpartikel bei einem zu geringen Wasseranteil verändern. Andererseits hat sich gezeigt, dass sich die Partikelgrößen bei einem zu hohen Wassergehalt ebenfalls verändern. Der optimale Wassergehalt kann für jede Substanz individuell bestimmt werden. Dabei hat sich gezeigt, dass die bevorzugte Wassermenge im Allgemeinen in dem Treibgas TG227 ea oder in Mischungen aus TG227 ea mit Treibgasen aus der Gruppe aus Propan, Butan, Pentan, Dimethylether, CHClF₂, CH₂F₂, CF₃CH₃, Isobutan, Isopentan und Neopentan, 10 bis 1000 ppm beträgt, besonders bevorzugt 50 bis 500 ppm und ganz besonders bevorzugt liegt die Wassermenge bei 100 bis 450 ppm.

Im Fall von Ipratropiumbromid-Monohydrat haltigen Formulierungen mit Treibgas TG227 ea beträgt der Wassergehalt der Formulierung zwischen 50 und 350 ppm.

Im Fall von Tiotropiumbromid-Monohydrat liegt der bevorzugte Wassergehalt bei vergleichbaren Größen wie für Ipratropiumbromid-Monohydrat. Der am stärksten bevorzugte Bereich liegt zwischen 50 und 230 ppm.

Erfindungsgemäß werden den Treibgasen oder den fertigen Aerosolsuspensionen bevorzugt diese Mengen Wasser zugesetzt, wenn das Treibgas, Treibgasgemisch oder die Formulierung bis auf das chemisch an den Wirkstoff gebundene Wasser kein sonstiges Wasser enthält (freies Wasser). Verfahrenstechnisch kann dabei das Wasser bereits dem Treibgas zugemischt werden, bevor die Arzneimittelsuspension hergestellt wird oder zunächst wird die Arzneimittelsuspension mit wasserfreiem Treibgas oder Treibgasgemisch hergestellt und anschließend wird die entsprechende Menge Wasser beigemischt.
Die ppm-Angaben beziehen sich als Bezugsgröße auf das verflüssigte Treibmittel.

Gegebenenfalls wird im Rahmen der vorliegenden Erfindung statt des Begriffs Suspension auch der Begriff Suspensionsformulierung verwendet. Beide Begriffe sind im Rahmen der vorliegenden Erfindung als gleichbedeutend anzusehen.

Die erfindungsgemäßem treibgashaltigen Inhalationsaerosole bzw. Suspensionsformulierungen können ferner weitere Bestandteile wie oberflächenaktive Mittel (Tenside, Surfactants), Adjuvantien, Antioxidantien oder Geschmacksmittel enthalten.

Die in den erfindungsgemäßen Suspensionen gegebenenfalls enthaltenen oberflächenaktiven Mittel (Tenside, Surfactants) sind vorzugsweise ausgewählt aus der Gruppe bestehend aus Polysorbat 20, Polysorbat 80, Myvacet 9-45, Myvacet 9-08, Isopropylmyristat , Ölsäure, Propylenglycol, Polyethylenglycol, Brij, Ethyloleat, Glyceryltrioleat Glycerylmonolaurat, Glycerylmonooleat, Glycerylmonosterat, Glycerylmonoricinoleate, Cetylalcohol, Oleyloleat, Sterylalkohol, Cetylpyridinumchlorid, Blockpolymere, natürliches Öl, Ethanol und Isopropanol. Von den vorstehend genannten Suspensionshilfsstoffen gelangen bevorzugt zur Anwendung Polysorbat 20, Polysorbat 80, Myvacet 9-45, Myvacet 9-08 oder Isopropylmyristat. Besonders bevorzugt gelangen zur Anwendung Myvacet 9-45 oder Isopropylmyristat.
Sofern in den erfindungsgemäßen Suspensionen oberflächenaktive Mittel enthalten sind, werden diese vorzugsweise in einem Anteil von 0,0005 - 1 %, besonders bevorzugt 0,005 - 0,5 % eingesetzt.

Die in den erfindungsgemäßen Suspensionen gegebenenfalls enthaltenen Adjuvantien sind vorzugsweise ausgewählt aus der Gruppe bestehend aus Alanin, Albumin, Ascorbinsäure, Aspartam, Betain, Cystein, Phosphorsäure, Salpetersäure, Salzsäure, Schwefelsäure und Zitronensäure. Besonders bevorzugt gelangen dabei zur Anwendung Ascorbinsäure, Phosphorsäure, Salzsäure oder Zitronensäure, besonders bevorzugt Salzsäure oder Zitronensäure.
Sofern in den erfindungsgemäßen Suspensionen Adjuvantien enthalten sind, werden diese vorzugsweise in einem Anteil von 0,0001-1,0 %, bevorzugt 0,0005-0,1 %, besonders bevorzugt 0,001-0,01 % eingesetzt, wobei ein Anteil von 0,001-0,005 % erfindungsgemäß von besonderer Bedeutung ist.

Die in den erfindungsgemäßen Suspensionen gegebenenfalls enthaltenen Antioxidantien sind vorzugsweise ausgewählt aus der Gruppe bestehend aus Ascorbinsäure, Zitronensäure, Natriumedetat, Editinsäure, Tocopherolen, Butylhydroxytoluol, Butylhydroxyanisol und Ascorbylpalmitat, wobei Tocopherole, Butylhydroxytoluol, Butylhydroxyanisol oder Ascorbylpalmitat bevorzugt zum Einsatz gelangen.

Die in den erfindungsgemäßen Suspensionen gegebenenfalls enthaltenen Geschmacksmittel sind vorzugsweise ausgewählt aus der Gruppe bestehend aus Pfefferminz, Sacharin, Dentomint^{®}, Aspartam und etherischen Ölen (beispielsweise Zimt, Anis, Menthol, Campher), wobei beispielsweise Pfefferminz oder Dentomint^{®} besonders bevorzugt sind.

Im Hinblick auf die inhalative Applikation ist es erforderlich, den Wirkstoff in feinteiliger Form bereitzustellen. Der Wirkstoff wird dazu entweder gemahlen (mikronisiert) oder über andere technische, im Stand der Technik prinzipiell bekannte Verfahren (beispielsweise Präzipitation, Sprühtrocknung) in feinteiliger Form gewonnen.
Verfahren zur Mikronisierung von Wirkstoffen sind im Stand der Technik bekannt. Vorzugsweise weist der Wirkstoff nach Mikronisierung eine mittlere Teilchengröße von 0,5 bis 10µm, bevorzugt von 1 bis 6µm, besonders bevorzugt von 1,5 bis 5µm auf. Vorzugsweise weisen wenigstens 50%, bevorzugt wenigstens 60%, besonders bevorzugt wenigstens 70% der Wirkstoffteilchen eine Teilchengröße auf, die innerhalb der vorstehend genannten Größenbereiche liegt. Besonders bevorzugt liegen wenigstens 80%, höchst bevorzugt wenigstens 90% der Wirkstoffteilchen mit ihrer Partikelgröße in den vorstehend genannten Bereichen.

Überraschenderweise wurde gefunden, dass ebenfalls Suspensionen dargestellt werden können, die neben den genannten Treibgasen lediglich den Wirkstoff bzw. die Wirkstoffe und keine weiteren Zusätze enthalten. Dementsprechend zielt ein weiterer Aspekt der vorliegenden Erfindung auf Suspensionen, die lediglich den Wirkstoff bzw. die Wirkstoffe ohne weitere Zusätze enthalten.

Zur Herstellung der erfindungsgemäßen Suspensionen kann nach im Stand der Technik bekannten Verfahren vorgegangen werden. Hierzu werden die Bestandteile der Formulierung mit dem oder den Treibgasen (ggf. bei niedrigen Temperaturen) gemischt und in geeignete Behälter abgefüllt.

Die vorstehend genannten erfindungsgemäßen treibgashaltigen Suspensionen können mittels im Stand der Technik bekannten Inhalatoren (pMDIs = pressurized metered dose inhalers) appliziert werden. Dementsprechend betrifft ein weiterer Aspekt der vorliegenden Erfindung Arzneimittel in Form von wie vorstehend beschriebenen Suspensionen in Verbindung mit einem oder mehreren zur Verabreichung dieser Suspensionen geeigneten Inhalatoren. Ferner betrifft die vorliegende Erfindung Inhalatoren, dadurch gekennzeichnet, dass sie vorstehend beschriebene erfindungsgemäße treibgashaltige Suspensionen enthalten.
Die vorliegende Erfindung betrifft ferner Behälter (z.B. Kartuschen), die ausgestattet sind mit einem geeigneten und vor der Verwendung bezüglich des Wassergehalts konditionierten Ventils. Die Behälter können in einem geeigneten Inhalator zur Anwendung gelangen und eine der vorstehend genannten erfindungsgemäßen treibgashaltigen Suspensionen enthalten. Geeignete Behälter (z.B. Kartuschen) und Verfahren zur Abfüllung dieser Kartuschen mit den erfindungsgemäßen treibgashaltigen Suspensionen sind aus dem Stand der Technik bekannt.

Aufgrund der pharmazeutischen Wirksamkeit von Anticholinergika betrifft die vorliegende Erfindung ferner die Verwendung der erfindungsgemäßen Suspensionen zur Herstellung eines inhalativ oder nasal applizierbaren Arzneimittels, bevorzugt zur Herstellung eines Arzneimittels zur inhalativen oder nasalen Behandlung von Erkrankungen, in denen Anticholinergika einen therapeutischen Nutzen entfalten können.

Besonders bevorzugt betrifft die vorliegende Erfindung ferner die Verwendung der erfindungsgemäßen Suspensionen zur Herstellung eines Arzneimittels zur inhalativen Behandlung von Atemwegserkrankungen, bevorzugt von Asthma oder COPD, Mukoviszidose, Cystische Fibrose; weiterhin von systemischen Erkrankungen, wie Schmerz, Migräne, Bluthochdruck, Erektionsstörungen.

Die nachfolgenden Beispiele dienen der exemplarischen, weitergehenden Illustration der vorliegenden Erfindung, ohne selbige auf deren Inhalt zu beschränken.

### Formulierungsbeispiele

| | |
|---|---|
| 1. | |
| - Ipratropiumbromid-Monohydrat | 0,03 Gew.% |
| - Fenoterol Hydrobromid | 0,07 Gew.% |
| - Myvacet Typ 9-08 (acetyliertes Monoglycerid) | 0,4 Gew.% |
| - TG 227ea | 99,5 Gew.% |
| **Gesamt** | **100 Gew: % (15,9 g)** |
| | |
| 2. | |
| - Ipratropiumbromid-Monohydrat | 0,03 Gew.% |
| - Fenoterol Hydrobromid | 0,07 Gew.% |
| - Isopropylmyristat | 0,4 Gew.% |
| - TG 227ea | 99,5 Gew.% |
| **Gesamt** | **100 Gew: % (15,9 g)** |
| | |
| 3. | |
| - Ipratropiumbromid-Monohydrat | 0,03 Gew.% |
| - Fenoterol Hydrobromid | 0,07 Gew.% |
| - Tween 20 | 0,4 Gew.% |
| - TG 227ea | 99,5 Gew.% |
| **Gesamt** | **100 Gew: % (15,9 g)** |
| | |
| 4. | |
| - Ipratropiumbromid-Monohydrat | 0,03 Gew.% |
| - Fenoterol Hydrobromid | 0,07 Gew.% |
| - Tween 80 | 0,4 Gew.% |
| - TG 227ea | 99,5 Gew.% |
| **Gesamt** | **100 Gew: % (15,9 g)** |
| | |
| 5. | |
| - Ipratropiumbromid-Monohydrat | 0,03 Gew.% |
| - Fenoterol Hydrobromid | 0,07 Gew.% |
| - Isopropylmyristat | 10,00 Gew.% |
| - TG 227ea | 89,90 Gew.% |
| **Gesamt** | **100 Gew: % (15,9 g)** |
| 6. | |
| - Ipratropiumbromid-Monohydrat | 0,03 Gew.% |
| - Fenoterol Hydrobromid | 0,07 Gew.% |
| - Isopropylmyristat | 10,00 Gew.% |
| - Sojalecithin | 0,004 Gew.% |
| - TG 227ea | 89,9 Gew.% |
| **Gesamt** | **100 Gew: % (15,9 g)** |
| | |
| 7. | |
| - Ipratropiumbromid-Monohydrat | 0,03 Gew.% |
| - Salbutamolsulfat | 0,19 Gew.% |
| - Tween 20 | 0,4 Gew.% |
| - TG 227ea | 99,38 Gew.% |
| **Gesamt** | **100 Gew: % (15,9 g)** |
| | |
| 8. | |
| - Ipratropiumbromid-Monohydrat | 0,03 Gew.% |
| - Salbutamolsulfat | 0,19 Gew.% |
| - TG 227ea | 99,78 Gew.% |
| **Gesamt** | **100 Gew: % (15,9 g)** |

Die Formulierungsbeispiele 1 bis 8 enthalten bevorzugt zwischen 50 und 300 ppm Wasser.

## Patentansprüche

1. Treibmittelhaltige Aerosolsuspension, enthaltend Wirkstoffpartikel mit chemisch gebundenem Wasser, wenigstens 85 Gew.% eines Treibgases, wobei das Treibgas TG 227 ea ist, gegebenfalls im Gemisch mit wenigstens einem weiterem Treibgas ausgewählt aus der Gruppe bestehend aus Propan, Butan, Pentan, Dimethylether, CHClF₂, CH₂F₂, CF₃CH₃, Isobutan, Isopentan und Neopentan, **dadurch gekennzeichnet, dass** die Aerosolsuspension neben dem chemisch an den Wirkstoff gebundenem Wasser weiteres, freies Wasser enthält, wobei die Suspension Ipratropiumbromid-Monohydrat oder Tiotropiumbromid-Monohydrat enthält, und die Menge an Wasser im Fall von Ipratropiumbromid-Monohydrat zwischen 50 und 350 ppm und im Fall von Tiotropiumbromid-Monohydrat zwischen 50 und 230 ppm liegt.

2. Aerosolsuspension nach Anspruch 1, **dadurch gekennzeichnet, dass** sie kristalline Wirkstoffpartikel enthält, die das Wasser als Kristallwasser, Hydrat oder Komplex binden.

3. Aerosolsuspension nach Anspruch 1 oder Anspruch 2, **dadurch gekennzeichnet, dass** die Menge an Wirkstoff zwischen 0,001 und 5 %, vorzugsweise 0,002 bis 3 %, insbesondere 0,002 bis 2 % liegt.

4. Aerosolsuspension nach einem der vorangegangenen Ansprüche, **dadurch gekennzeichnet, dass** sie als weitere Bestandteile oberflächenaktive Mittel (Tenside, Surfactants), Adjuvantien, Antioxidantien und/oder Geschmacksmittel enthalten.

5. Aerosolsuspension nach einem der vorangegangenen Ansprüche, **dadurch gekennzeichnet, dass** sie als oberflächenaktive Mittel (Tenside, Surfactants) eine oder mehrere Verbindungen ausgewählt aus der Gruppe bestehend aus Polysorbat 20, Polysorbat 80, Myvacet 9-45, Myvacet 9-08, Isopropylmyristat, Ölsäure, Propylenglycol, Polyethylenglycol, Brij, Ethyloleat, Glyceryltrioleat Glycerylmonolaurat, Glycerylmonooleat, Glycerylmonosterat, Glycerylmonoricinoleate, Cetylalcohol, Oleyloleat, Sterylalkohol, Cetylpyridinumchlorid, Blockpolymere, natürliches Öl, Ethanol und Isopropanol enthalten.

6. Aerosolsuspension nach einem der vorangegangenen Ansprüche, **dadurch gekennzeichnet, dass** sie als Adjuvantien eine oder mehrere Verbindungen ausgewählt aus der Gruppe bestehend aus Alanin, Albumin, Ascorbinsäure, Aspartam, Betain, Cystein, Phosphorsäure, Salpetersäure, Salzsäure, Schwefelsäure und Zitronensäure enthalten.

7. Aerosolsuspension nach einem der vorangegangenen Ansprüche, **dadurch gekennzeichnet, dass** sie als Antioxidantien eine oder mehrere Verbindungen ausgewählt aus der Gruppe bestehend aus Ascorbinsäure, Zitronensäure, Natriumedetat, Editinsäure, Tocopherolen, Butylhydroxytoluol, Butylhydroxyanisol und Ascorbylpalmitat enthalten.

8. Aerosolsuspension nach einem der vorangegangenen Ansprüche, **dadurch gekennzeichnet, dass** sie neben dem Wirkstoff, Wasser und dem oder den Treibgasen keine weiteren Bestandteile enthalten.

9. Verwendung einer Aerosolsuspension gemäß einem der Ansprüche 1 bis 8 zur Herstellung eines Arzneimittels, bevorzugt zur Herstellung eines Arzneimittels zur inhalativen oder gegebenenfalls nasalen Behandlung von Erkrankungen, in denen Anticholinergika einen therapeutischen Nutzen entfalten können.

10. Verwendung nach Anspruch 9, **dadurch gekennzeichnet, dass** es sich bei den Erkrankungen um Atemwegserkrankungen, bevorzugt um Asthma, COPD, Mucoviszidose oder cystische Fibrose handelt.

11. Verfahren zur Herstellung von Aerosolsuspensionen gemäß einem der Ansprüche 1 bis 8, **dadurch gekennzeichnet, dass** wasserhaltiges Treibgas oder Treibgasgemisch eingesetzt wird, um die Aerosolsuspension herzustellen.

12. Verfahren zur Herstellung von Aerosolsuspensionen gemäß einem der Ansprüche 1 bis 8, **dadurch gekennzeichnet, dass** mit wasserfreiem Treibgas oder Treibgasgemisch eine Aerosolsuspension hergestellt wird und anschließend Wasser beigemischt wird.

## Claims

1. Propellant-containing aerosol suspension containing particles of active substance with chemically bound water, at least 85 wt.% of a propellant gas, wherein the propellant gas is TG 227 ea, optionally in admixture with at least one other propellant gas selected from among propane, butane, pentane, dimethylether, CHClF₂, CH₂F₂, CF₃CH₃, isobutane, isopentane and neopentane, **characterised in that** the aerosol suspension contains additional free water in addition to the water chemically bound to the active substance, the suspension containing ipratropium bromide monohydrate or tiotropium bromide monohydrate, and the quantity of water being between 50 and 350 ppm in the case of ipratropium bromide monohydrate and being between 50 and 230 ppm in the case of tiotropium bromide monohydrate.

2. Aerosol suspension according to claim 1, **characterised in that** it contains crystalline particles of active substance which bind the water as water of crystallisation, hydrate or complex.

3. Aerosol suspension according to claim 1 or claim 2, **characterised in that** the amount of active substance is between 0.001 and 5 %, preferably 0.002 to 3 %, particularly 0.002 to 2 %.

4. Aerosol suspension according to one of the preceding claims, **characterised in that** it contains as further ingredients surface-active substances (surfactants), adjuvants, antioxidants and/or flavourings.

5. Aerosol suspension according to one of the preceding claims, **characterised in that** it contains as surface-active substances (surfactants) one or more compounds selected from among Polysorbate 20, Polysorbate 80, Myvacet 9-45, Myvacet 9-08, isopropyl myristate, oleic acid, propyleneglycol, polyethyleneglycol, Brij, ethyl oleate, glyceryl trioleate, glyceryl monolaurate, glyceryl monooleate, glyceryl monostearate, glyceryl monoricinoleate, cetylalcohol, oleyloleate, sterylalcohol, cetylpyridinium chloride, block polymers, natural oil, ethanol and isopropanol.

6. Aerosol suspension according to one of the preceding claims, **characterised in that** it contains as adjuvants one or more compounds selected from among alanine, albumin, ascorbic acid, aspartame, betaine, cysteine, phosphoric acid, nitric acid, hydrochloric acid, sulphuric acid and citric acid.

7. Aerosol suspension according to one of the preceding claims, **characterised in that** it contains as antioxidants one or more compounds selected from the group consisting of ascorbic acid, citric acid, sodium edetate, editic acid, tocopherols, butylhydroxytoluene, butylhydroxyanisol and ascorbylpalmitate.

8. Aerosol suspension according to one of the preceding claims, **characterised in that** it contains no other ingredients in addition to the active substance, water and the propellant gas or gases.

9. Use of an aerosol suspension according to one of claims 1 to 8 for preparing a medicament, preferably for preparing a medicament for the treatment, by inhalation or optionally by nasal route, of diseases in which anticholinergics may confer a therapeutic benefit.

10. Use according to claim 9, **characterised in that** the diseases are respiratory diseases, preferably asthma, COPD, mucoviscidosis or cystic fibrosis.

11. Process for preparing aerosol suspensions according to one of claims 1 to 8, **characterised in that** water-containing propellant gas or propellant gas mixture is used to prepare the aerosol suspension.

12. Process for preparing aerosol suspensions according to one of claims 1 to 8, **characterised in that** an aerosol suspension is prepared with anhydrous propellant gas or propellant gas mixture and then water is added.

## Revendications

1. Suspension aérosol contenant un agent propulseur comportant des particules de substance active avec de l'eau liée chimiquement, au moins 85 % en poids d'un gaz propulseur, le gaz propulseur TG 227 ea étant éventuellement en mélange avec au moins un autre gaz propulseur choisi dans le groupe comprenant le propane, le butane, le pentane, le diméthyléther, le CHClF₂, le CH₂F₂, le CF₃CH₃, l'isobutane, l'isopentane et le néopentane, **caractérisée en ce que** la suspension aérosol contient, outre l'eau liée chimiquement à la substance active, une autre eau libre, la suspension contenant du monohydrate de bromure d'ipratropium ou du monohydrate de bromure de tiotropium et la quantité d'eau se situant, pour le monohydrate de bromure d'ipratropium, entre 50 et 350 ppm, et pour le monohydrate de bromure de tiotropium, entre 50 et 230 ppm.

2. Suspension aérosol selon la revendication 1, **caractérisée en ce qu'**elle contient des particules de substance active cristallines qui lient l'eau en tant qu'eau de cristallisation, hydrate ou complexe.

3. Suspension aérosol selon la revendication 1 ou la revendication 2, **caractérisée en ce que** la quantité de substance active se situe entre 0,001 et 5 %, de préférence 0,002 à 3 %, en particulier 0,002 à 2 %.

4. Suspension aérosol selon l'une quelconque des revendications précédentes, **caractérisée en ce qu'**elle contient comme autres composants, des tensioactifs (agents tensioactifs, surfactants), des adjuvants, des antioxydants et/ou des aromatisants.

5. Suspension aérosol selon l'une quelconque des revendications précédentes, **caractérisée en ce qu'**elle contient en tant que tensioactif (agents tensioactifs, surfactants) un ou plusieurs composés choisis dans le groupe comprenant le Polysorbate 20, le Polysorbate 80, le Myvacet 9-45, le Myvacet 9-08, le myristate d'isopropyle, l'acide oléique, le propylène glycol, le polyéthylène glycol, le Brij, l'oléate d'éthyle, le trioléate de glycéryle, le monolaurate de glycéryle, le mono-oléate de glycéryle, le monostéarate de glycéryle, le monoricinoléate de glycéryle, l'alcool cétylique, l'oléate d'oléyle, l'alcool stérylique, le chlorure de cétylpyridinium, les polymères en bloc, l'huile naturelle, l'éthanol et l'isopropanol.

6. Suspension aérosol selon l'une quelconque des revendications précédentes, **caractérisée en ce qu'**elle contient comme adjuvants, un ou plusieurs composés choisis dans le groupe comprenant l'alanine, l'albumine, l'acide ascorbique, l'aspartame, la bétaïne, la cystéine, l'acide phosphorique, l'acide nitrique, l'acide chlorhydrique, l'acide sulfurique et l'acide citrique.

7. Suspension aérosol selon l'une quelconque des revendications précédentes, **caractérisée en ce qu'**elle contient comme antioxydants, un ou plusieurs composés choisis dans le groupe comprenant l'acide ascorbique, l'acide citrique, l'édétate de sodium, l'acide éditique, les tocophérols, le butylhydroxytoluène, le butylhydroxyanisol et le palmitate d'ascorbyle.

8. Suspension aérosol selon l'une quelconque des revendications précédentes, **caractérisée en ce que**, outre la substance active, l'eau et le ou les gaz propulseur (s), elle ne contient pas d'autres composants.

9. Utilisation d'une suspension aérosol selon l'une des revendications 1 à 8, pour la production d'un médicament, de préférence pour la production d'un médicament pour le traitement par inhalation ou éventuellement le traitement nasal de maladies, dans lesquelles les anticholinergiques peuvent développer un avantage thérapeutique.

10. Utilisation selon la revendication 9, **caractérisée en ce que** les maladies sont des maladies des voies respiratoires, de manière préférée l'asthme, la BPCO, la mucoviscidose ou la fibrose kystique.

11. Procédé de production de suspensions aérosols selon l'une quelconque des revendications 1 à 8, **caractérisé en ce que** le gaz propulseur contenant de l'eau ou le mélange de gaz propulseurs est utilisé pour préparer la suspension aérosol.

12. Procédé de production de suspensions aérosols selon l'une quelconque des revendications 1 à 8, **caractérisé en ce qu'**une suspension aérosol est produite avec un gaz propulseur ou un mélange de gaz propulseurs anhydres et ensuite, on ajoute de l'eau.
